# EUROPÄISCHE PATENTSCHRIFT

(11) **EP 3 108 842 B1**
(45) Veröffentlichungstag und Bekanntmachung des Hinweises auf die Patenterteilung: **27.03.2019**
(21) Anmeldenummer: 16175470.0
(22) Anmeldetag: 21.06.2016
(51) Int. Cl.: A61B 50/30

(54) **VERPACKUNGSANORDNUNG UND VERFAHREN ZUR HERSTELLUNG EINER VERPACKUNG FÜR EIN STERILISIERBARES PRODUKT**
PACKAGING ASSEMBLY AND METHOD FOR MANUFACTURING PACKAGING FOR A STERILIZABLE PRODUCT
SYSTEME D'EMBALLAGE ET PROCEDE DE FABRICATION D'UN EMBALLAGE POUR UN PRODUIT STERILISABLE

(30) Priorität: 25.06.2015 DE 102015008288
(43) Veröffentlichungstag der Anmeldung: 28.12.2016
(73) Patentinhaber: steripac GmbH, 75365 Calw (DE)
(72) Erfinder: Hutzler, Martin, 75365 Calw (DE)
(74) Vertreter: Wacker, Jost Oliver

(56) Entgegenhaltungen:
- US-A- 4 321 781
- US-A1- 2009 272 664
- US-A1- 2015 021 221
- US-B1- 6 622 864

## Beschreibung

Die Erfindung betrifft eine Verpackungsanordnung für die Aufnahme eines zu sterilisierenden Produktes, wie insbesondere eines medizinischen Produktes, nach dem Oberbegriff des Anspruchs 1 sowie eine hieraus erstellte Verpackung und ein Verfahren zur Herstellung der Verpackung. Die Verpackungsanordnung weist dabei ein blisterförmiges erstes Verpackungselement mit wenigstens einem Aufnahmeraum auf. In diesen kann ein zweites Verpackungselement der Verpackungsanordnung eingesetzt werden, das zur positionsstabilen Fixierung des Produktes dient. Zudem weist die Verpackungsanordnung ein Schließelement auf, das unter Zwischenlage des zweiten Verpackungselementes über einen Bundbereich mit dem ersten Verpackungselement luftdicht verbunden werden kann, um dieses zu verschließen. Ferner ist an dem ebenfalls bei üblicher Umgebungstemperatur im Wesentlichen formstabil beziehungsweise starr ausgebildeten zweiten Verpackungselement ein Verformungsbereich vorgesehen, zwischen dem und einem Aufnahmeboden des Aufnahmeraumes ein Klemmbereich für das Produkt ausgebildet werden kann. Dabei ist der Verformungsbereich bei einer gegenüber einer üblichen Umgebungs- beziehungsweise Raumtemperatur erhöhter Verformungstemperatur von 90 bis 140°C plastisch verformbar. Zudem weist das zweite Verpackungselement einen sich um den Verformungsbereich umlaufend erstreckenden und diesen dabei aufspannenden Steckbereich auf, der an einem Rand des ersten Verpackungselementes formschlüssig festlegbar ist und hierzu bereichsweise auf die Abmessungen des Randes passgenau abgestimmt ist. Derartige Verpackungsanordnungen können insbesondere zur sterilen Verpackung unterschiedlich geformter Produkte verwendet werden.

US 2009/0272664 A1 beschreibt eine Verpackungsanordnung zur sterilen und schützenden Aufnahme von medizinischen Instrumenten. Hierzu weist die Verpackungsanordnung zwei ineinander steckbare blisterförmige Verpackungselemente auf, deren Boden jeweils durch ein elastisches Folienmaterial gebildet ist. Beim Ineinanderstecken der Verpackungselemente wird dadurch ein zuvor dazwischen aufgenommenes Instrument zwischen beiden Folien gehalten, die sich dabei an die Form des Instrumentes anpassen. Anschließend wird die Verpackungsanordnung zur Sicherstellung der sterilen Aufbewahrung in einer Umhüllung aufgenommen.

US 6,622,864 B1 beschreibt eine Verpackungsanordnung zur sterilen Aufnahme von Gewebeproben. Um hierbei eine Migration von Fetten beziehungsweise lipiden Spurenelementen in Verbindungsbereiche der Verpackungen zu vermeiden, die eine Undichtigkeit zur Folge haben können, ist vorgesehen eine innere peelbare Blisterverpackung in einer äußeren peelbaren Blisterverpackung aufzunehmen und dabei beide Blisterverpackungen aus einem porenfreien Material herzustellen.

US 2015/0021221 A1 beschreibt ein mehrteiliges Verpackungssystem für medizinische Geräte, die teilweise vor ihrem Gebrauch befüllt werden müssen. Hierzu weist das Verpackungssystem ein erstes Verpackungselement auf, das in ein zweites Verpackungselement eingesetzt werden kann, wobei die medizinischen Geräte im Verpackungszustand zwischen beiden Verpackungselementen gehalten sind. Das erste Verpackungselement kann nach dem Entfernen der medizinischen Geräte zudem derart umgeformt und in das zweite Verpackungselement eingesetzt werden, dass es eine Art Ständer für das zu befüllende Gerät dient. Ferner ist eine Umverpackung zur verschlossenen Aufnahme der beiden Verpackungselemente vorgesehen, die eine Schale aufweist, die durch eine Schließfolie geschlossen werden kann.

Aus DE 10 2013 004 168 A1 ist eine Verpackungsanordnung und ein Verfahren zum Verpacken von sterilen Operationswerkzeugen bekannt. Diese weist eine erste Schutzverpackung auf, an der ein betreffendes Operationswerkzeug festgeklemmt werden kann. Diese erste Schutzverpackung wird dann zusammen mit dem daran befestigten Operationswerkzeug in eine Blisterverpackung eingesetzt, die durch eine Siegelfolie verschließbar ist. Im fertig verpackten Zustand ist das Operationswerkzeug dabei durch die Schutzverpackung gegenüber der Blisterverpackung positionsstabil gelagert.

Nachteilig an dieser bekannten Verpackungsanordnung ist, dass diese lediglich für Produkte verwendet werden kann, die einen an die Klemmmittel der Schutzverpackung angepassten Abschnitt aufweisen, wie beispielsweise einen Griffabschnitt eines Operationswerkzeuges mit einer vorgegebenen Breite. Darüber hinaus sind die Herstellungskosten für derartige Verpackungsanordnungen insbesondere aufgrund der benötigten Klemmmittel an der Schutzverpackung relativ hoch.

Insbesondere bei sehr kleinen Stückzahlen eines Produkttyps ist diese Form von relativ exakt angepassten Verpackungen somit oftmals aufgrund von z.B. hohen Werkzeugkosten wirtschaftlich nicht anwendbar. Typische Produkte für eine große Variantenvielfalt aufgrund unterschiedlicher Größen oder Geometrien sind hierbei beispielsweise Hüftgelenke, Dentalimplantate, Knochenschrauben, Knochenplatten, Wirbelsäulenimplantate und Ähnliches. Hinzu kommen aufgrund von neuen technischen Möglichkeiten, wie zum Beispiel Lasersintern, Produkte, insbesondere Implantate, die als Einzelstück patientenbezogen auf dessen Körperbau hergestellt werden. Diese Einzelstücke können sich in Form, Struktur, Größe oder Gewicht von Produkt zu Produkt unterscheiden. Für solche Produkte sind exakt ausgelegte Verpackungen, die die Produktgeometrie aufnehmen, um eine optimale Lagerung zu gewährleisten, weder in kostenmäßiger noch in zeitlicher Hinsicht in einem angemessenen Rahmen herstellbar.

Neben den oben beschriebenen Verpackungsanordnungen sind heute ferner sogenannte Flachbeutel bekannt, bei denen das Produkt in einen flexiblen Beutel gelegt wird, welcher anschließend versiegelt wird. Hierbei kann auch ein Vakuum im Beutel hergestellt werden, wodurch eine Bewegung des Produktes innerhalb des Beutels verhindert wird.

Diese Technik ist auch für Einzelchargen anwendbar. Nachteilig hierbei sind jedoch neben einer wenig gefälligen Optik vor allem die Gefahren einer Verpackungsbeschädigung durch eckige oder gar scharfkantige Produkte, da die bekannten Flachbeutel in der Regel aus relativ dünnen flexiblen Materialien hergestellt sind, die nur bedingt einer mechanischen Belastung widerstehen können.

Die Aufgabe der Erfindung ist es, bei einer gattungsgemäße Verpackungsanordnung die genannten Nachteile zu vermeiden und bei niedrigen Herstellungskosten eine Verpackung zur Verfügung zu stellen, die eine sichere Aufnahme von in Form, Struktur, Größe und Gewicht relativ unterschiedlichen Produkten ermöglicht.

Diese Aufgabe wird durch eine fertig gestellte Verpackung mit einer Verpackungsanordnung und dem darin aufgenommenen zu sterilisierenden Produkt mit den Merkmalen des Anspruchs 1 gelöst. Dabei ist der Aufnahmeraum durch wenigstens einen starren Tiefziehbereich des ersten Verpackungselementes gebildet. Zudem liegt der Verformungsbereich des zweiten Verpackungselementes in einem Kontaktbereich zwischen dem Produkt und dem zweiten Verpackungselement an einer Oberfläche des Produktes an und ist komplementär zu dieser plastisch verformt sowie bei üblicher Umgebungstemperatur mit dieser Formgebung ausgehärtet. Auf diese Weise kann beim Einsetzen des zweiten Verpackungselementes in das erste Verpackungselement eine gegenseitig positionsstabile Festlegung beider Elemente erreicht werden. Gleichzeitig kann hierbei durch den Verformungsbereich das zweite Verpackungselement durch das zu verpackende Produkt selbst und im Zuge des Verpackungsprozesses an dessen Form, Struktur und Größe angepasst werden. Dabei passt sich der erwärmte Verformungsbereich beim Anlegen an das jeweilige Produkt in dem Kontaktbereich an dessen Form und Größe an. Sobald das zweite Verpackungselement dann nicht mehr die erhöhte Verformungstemperatur aufweist, härtet der Verformungsbereich mit der an das Produkt angepassten Formgebung aus und legt dieses dauerhaft in dem Klemmbereich beziehungsweise gegenüber dem ersten Verpackungselement fest. Auf diese Weise können die gleichen Verpackungsanordnungen zur sicheren Verpackung einer besonders großen Vielzahl unterschiedlichster zu sterilisierender Produktformen verwendet werden, wobei die positionsstabile Lagerung mit kostengünstigen Mitteln erfolgt. Hierdurch ermöglicht die Verpackungsanordnung insbesondere auch bei kleinen Stückzahlen oder Einzelstücken eine wirtschaftliche Herstellung von produktbezogenen und produktangepassten Verpackungen sowie von individuell angepassten Einzelverpackungen. Dabei können in einer derartigen Verpackungsanordnung problemlos auch zwei oder mehrere Produkte gleichzeitig aufgenommen und fixiert werden. Um hierbei während der Erwärmung des Verformungsbereichs auf die Verformungstemperatur eine gleichzeitige Verformung des Steckbereiches zu vermeiden, wird die Erwärmung im Wesentlichen auf den Verformungsbereich beschränkt. Alternativ oder zusätzlich hierzu kann der Steckbereich auch derart ausgebildet sein, dass er gegenüber dem Verformungsbereich eine deutlich höher liegende plastische Verformungstemperatur aufweist. Die Verpackung und das darin aufgenommene Produkt können somit gemeinsam einer Sterilisation unterzogen werden, wie beispielsweise durch Beaufschlagung mit Gamma-Strahlung oder durch jedes andere bekannte Sterilisationsverfahren, das geeignet ist, um auch das bereits in der Verpackung aufgenommene Produkt zu sterilisieren. Eine solche Verpackung kann dabei sowohl eine Beschädigung als auch eine Verunreinigung des Produktes, insbesondere in Folge einer Relativbewegung gegenüber der Verpackung, wirksam verhindern. Hierbei kann insbesondere verhindert werden, dass das Produkt die Verpackung im Zuge einer Relativbewegung durchstößt und dabei beschädigt oder verunreinigt wird. Zudem kann das Produkt mittels einer solchen Verpackung gegen mechanische Beanspruchungen von außen geschützt werden, die beispielsweise bei seiner Handhabung, beim Transport oder einer unzureichenden Lagerung des verpackten Produktes auftreten können.

Dabei ist es günstig, wenn auch das zweite Verpackungselement blisterförmig ausgebildet ist, so dass es bei Anlage des Steckbereiches am Rand des ersten Verpackungselementes mit dem Verformungsbereich relativ weit in den Aufnahmeraum hinein ragt. Auf diese Weise kann der Verformungsbereich über einen relativ großen Bereich mit dem darin aufgenommenen Produkt in Anlage kommen und dabei an dessen Form angepasst werden.

Alternativ hierzu ist es auch möglich, das zweite Verpackungselement flach auszubilden, um beispielsweise eine kostengünstigere Herstellung der Verpackungsanordnung zu ermöglichen. In diesem Fall erstecken sich der Verformungsbereich und der diesen aufspannende und ebenfalls flach ausgebildete Steckbereich entlang einer gemeinsamen Ebene.

Vorteilhafterweise sind das Schließelement und das erste Verpackungselement wenigstens in dem miteinander verbindbaren Bereich durch gegenseitig siegelfähige Kunststoff-Materialien gebildet. Dabei kann das erste Verpackungselement beispielsweise aus PET, PE oder PP und das Schließelement aus einer Verbundfolie mit einer entsprechenden außen liegenden Siegelschicht, aus PE oder aus einem Medizinpapier gebildet sein. Hierdurch kann das erste Verpackungselement mit dem darin aufgenommenen und durch das zweite Verpackungselement fixierten Produkt auf einfache Weise durch Beaufschlagung mit Druck und erhöhter Temperatur dicht verschlossen werden.

Zudem ist es günstig, wenn das erste Verpackungselement und das Schließelement über eine peelbare Verbindung beziehungsweise eine Peelschicht verbunden sind. Hierdurch kann die fertig gestellte Verpackung in besonders komfortabler Weise geöffnet werden, um das darin aufgenommene Produkt heraus nehmen zu können.

Vorteilhafterweise weist der Aufnahmeboden eine Produktauflage auf, die gegenüber dem übrigen Aufnahmeboden erhöht ausgebildet ist. Die erhöhte Produktauflage kann dabei beispielsweise zur Aufnahme eines relativ flachen Produktes dienen, um sicherzustellen, dass der Verformungsbereich beim Einsetzen des zweiten Verpackungselementes in den Aufnahmeraum über einen ausreichend großen Bereich der Oberfläche des Produktes mit diesem in Anlage kommt. Hierdurch ist auch bei flachen Produkten gewährleistet, dass der Verformungsbereich in ausreichendem Maße an die Form des Produktes angepasst wird, um eine sichere Fixierung desselben gegenüber dem ersten Verpackungselement zu erzielen.

Ferner wird die genannte Aufgabe durch ein Verfahren zur Herstellung einer solchen sterilisierbaren Verpackung gelöst, wobei in einem ersten Schritt das Produkt im Aufnahmeraum des ersten Verpackungselementes angeordnet und das zweite Verpackungselement auf die Verformungstemperatur erwärmt wird, bis der Verformungsbereich einen plastisch verformbaren Zustand erreicht. In einem zweiten Schritt wird das erwärmte zweite Verpackungselement dann in den Aufnahmeraum eingesetzt, wobei der Verformungsbereich durch Anlage an der Oberfläche des Produktes im Kontaktbereich komplementär zu diesem ausgeformt wird. In einem dritten Schritt wird ferner eine Aufnahmeöffnung des Aufnahmeraumes mittels des Schließelementes verschlossen, um die herum sich der Bundbereich erstreckt. Hierdurch kann die Verpackung mit dem aufgenommenen Produkt in einfacher Weise hergestellt und anschließend zusammen mit diesem als Einheit sterilisiert werden. Die erfindungsgemäße Vorgehensweise ermöglicht dabei die wirtschaftliche Herstellung einer sterilisierbaren Verpackung unterschiedlicher Produkte auch in kleinen Stückzahlen oder als Einzelverpackung individueller Produkte.

In einer weiteren vorteilhaften Ausführungsform wird im ersten Schritt lediglich der Verformungsbereich auf die Verformungstemperatur erwärmt. Hierdurch ist gewährleistet, dass der Steckbereich des zweiten Verpackungselementes insbesondere beim Einstecken des zweiten Verpackungselementes in den Aufnahmeraum eine ausreichend hohe Steifigkeit beibehält, um einen relativ großen Teil des Verformungsbereiches mit der Oberfläche des darin aufgenommenen Produktes in Anlage bringen zu können, damit sich dieser komplementär zu der Oberfläche ausformen kann. Zudem kann hierdurch ein stabiler und spielfreier Formschluss zwischen dem Steckbereich des zweiten Verpackungselementes und dem Rand des ersten Verpackungselementes gewährleistet werden.

Vorteilhafterweise wird die Verpackungsanordnung mit dem darin festgelegten Produkt in einem weiteren Schritt einer Sterilisation unterzogen. Hierdurch können die Verpackungsanordnung und das Produkt während und nach der Sterilisation als Einheit gehandhabt werden, wobei eine Relativbewegung des Produktes gegenüber der Verpackung unterbunden wird.

In den Figuren ist eine beispielhafte Ausführungsform der Erfindung dargestellt. Es zeigen:
- Figur 1: eine perspektivische Explosionsansicht einer erfindungsgemäßen Verpackungsanordnung,
- Figur 2: eine geschnittene Ansicht der Verpackungsanordnung nach Fig. 1 mit aufgenommenem Produkt,
- Figur 3: eine geschnittene Ansicht einer aus der Verpackungsanordnung nach Fig. 2 hergestellten Verpackung und
- Figur 4: eine geschnittene Ansicht einer aus einer alternativen Ausführungsform der Verpackungsanordnung hergestellten Verpackung mit einer erhöhten Produktaufnahme.

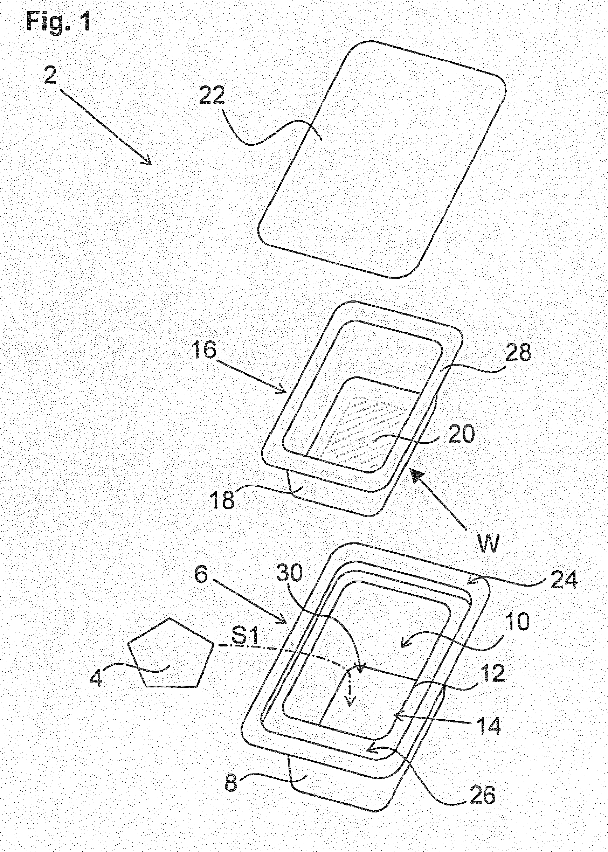

Fig. 1 zeigt eine Verpackungsanordnung 2 zur Aufnahme eines zu sterilisierenden medizinischen Produktes 4, wie beispielsweise einem Hüftgelenk, einem Dentalimplantat, einer Knochenschraube, einer Knochenplatte, einem Wirbelsäulenimplantat oder einem sonstigen, beispielsweise durch Lasersintern individuell hergestellten medizinischen Produkt. Hierfür weist die Verpackungsanordnung 2 ein erstes Verpackungselement 6 in Form eines aus Kunststoff, wie beispielsweise PET, PE oder PP, hergestellten bei üblicher Umgebungstemperatur im Wesentlichen starren Blisters mit einem Tiefziehbereich 8 auf, der einen Aufnahmeraum 10 begrenzt. Der Aufnahmeraum 10 weist dabei eine von einem Rand 12 umschlossene Aufnahmeöffnung 14 auf.

Über diese Aufnahmeöffnung 14 kann ein zweites Verpackungselement 16 in den Aufnahmeraum 10 eingesetzt werden. Dieses zweite Verpackungselement 16 ist dabei ebenfalls durch einen aus bei üblicher Umgebungstemperatur im Wesentlichen formstabilem beziehungsweise starrem Kunststoff hergestellten Blister gebildet, der einen tiefgezogenen Steckbereich 18 und einen sich innerhalb des Steckbereiches 18 erstreckenden Verformungsbereich 20 ausbildet. Der Verformungsbereich 20 besteht dabei aus einem Kunststoffmaterial, das bei Erwärmung auf eine vorgegebene Verformungstemperatur von beispielsweise 90 bis 140 ° Celsius plastisch verformbar ist, wie beispielsweise PET, PE oder PP. Demgegenüber ist hinsichtlich des Steckbereiches 18 vorgesehen, dass dieser während des gesamten Verpackungsprozesses im Wesentlichen formstabil bleibt. Dies kann während der Erwärmung des Verformungsbereiches 20 beispielsweise durch eine entsprechend begrenzte Wärmebeaufschlagung gemäß Pfeil W oder durch eine ausreichend wärmebeständige Materialauswahl sichergestellt werden.

Ferner weist die Verpackungsanordnung 2 ein Schließelement 22 auf, das mit einem Bundbereich 24 des ersten Verpackungselementes 6 verbunden werden kann. In diesen Bundbereich 24 kann dabei, wie dargestellt, eine Vertiefung 26 eingelassen sein, die komplementär zu einem umlaufenden Kragen 28 des Steckbereichs 18 ausgebildet ist. Hierdurch kann das zweite Verpackungselement 16 beim Einsetzten in den Aufnahmeraum 10 in eine exakt vorgegebene Endposition verbracht werden, in der der Kragen 28 positionsstabil in der Vertiefung 26 des Bundbereiches 24 angeordnet ist. Insbesondere nach einerzusätzlichen Sicherung durch das Schließelement 22, kann somit eine stabile und spielfreie Festlegung des zweiten Verpackungselementes 16 am ersten Verpackungselement 6 gewährleistet werden.

Um das Produkt 4 mittels der Verpackungsanordnung 2 zu verpacken, wird es in einem ersten Schritt gemäß Pfeil S1 an einem Aufnahmeboden 30 des Aufnahmeraumes 10 positioniert. Zudem wird der Verformungsbereich 20 mit Wärmeenergie W beaufschlagt, um diesen auf die Verformungstemperatur zu erwärmen, so dass er mittels mechanischer Beaufschlagung plastisch verformt werden kann.

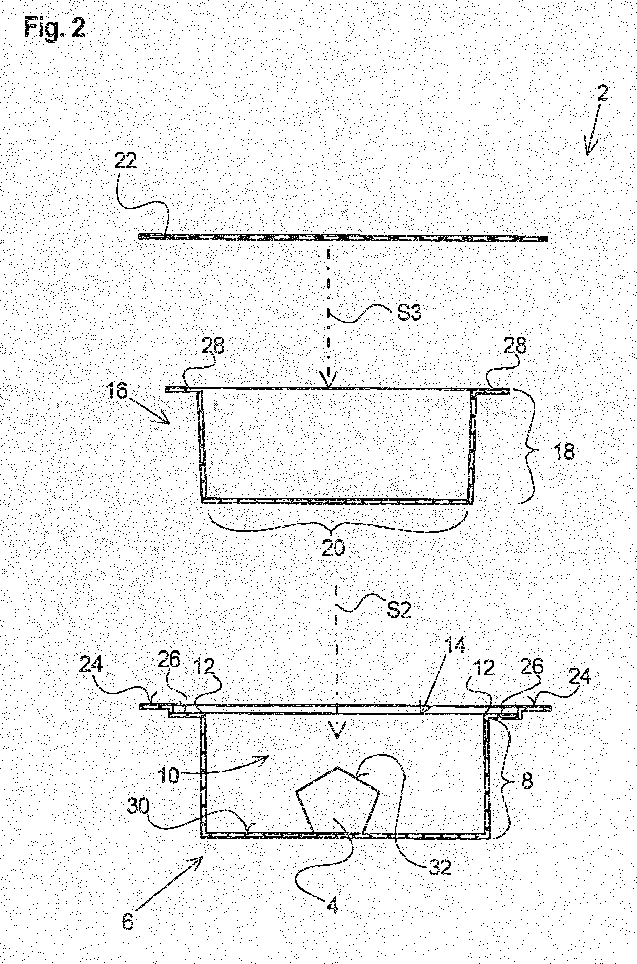

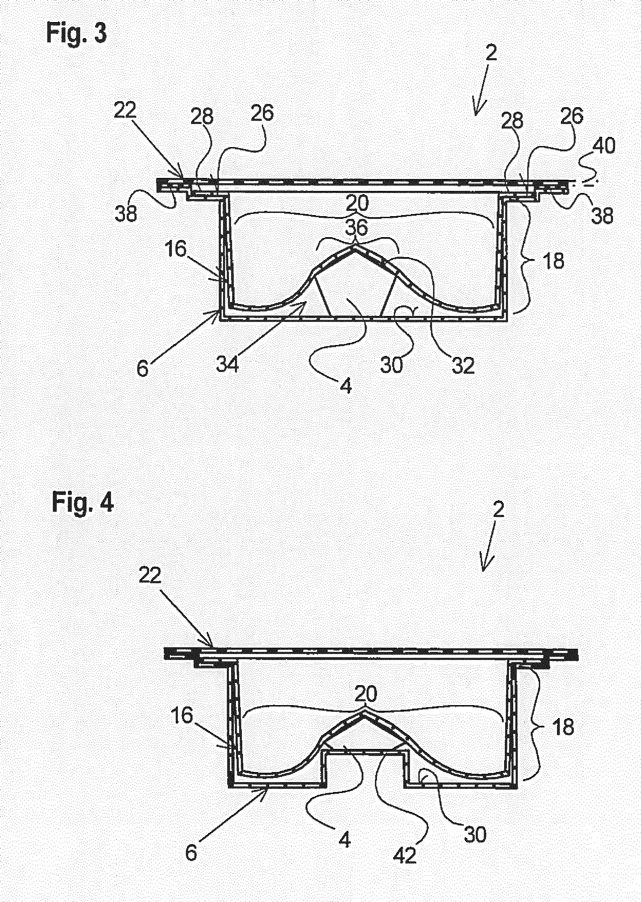

Wie durch Pfeil S2 in Fig. 2 angedeutet, wird dann das zweite Verpackungselement 16 in einem zweiten Schritt über die Aufnahmeöffnung 14 in den Aufnahmeraum 10 eingesetzt. Hierbei drückt der im Wesentlichen formstabile Steckbereich 18 den erwärmten Verformungsbereich 20, der innerhalb des Steckbereiches 18 aufgespannt ist, gegen eine Oberfläche 32 des Produktes 4 und bildet dabei zusammen mit dem Aufnahmeboden 30 einen Klemmbereich 34 aus, in dem das Produkt zwischen dem ersten Verpackungselement 6 und dem zweiten Verpackungselement 16 festgeklemmt wird, wie in Fig. 3 dargestellt. Das zweite Verpackungselement 16 wird dabei soweit in den Aufnahmeraum 10 hinein verlagert, bis der Kragen 28 an der Vertiefung 26 anliegt beziehungsweise der Steckbereich 18 mit dem Rand 12 der Aufnahmeöffnung 14 in formschlüssigem Eingriff steht.

Durch die hierbei erfolgende Anpressbewegung des erwärmten Verformungsbereiches 20 gegen die Oberfläche 32 des Produktes verformt sich dieser in jedem Fall derart, dass er insbesondere in einem Kontaktbereich 36 mit der Oberfläche 32 des Produktes 4 eine zu dieser im Wesentlichen komplementäre Form annimmt.

Sobald sich die Materialtemperatur des Verformungsbereiches 20 anschließend wieder einer üblichen Umgebungstemperatur annähert und wieder unter der Verformungstemperatur liegt, härtet er mit der neuen Formgebung aus und fixiert das Produkt 4 auf diese Weise dauerhaft in seiner Position gegenüber dem ersten Verpackungselement 6.

In einem dritten Schritt wird dann, wie in Fig. 2 durch Pfeil S3 angedeutet, das Schließelement 22 mit dem Bundbereich 24 umlaufend verbunden, um das im Aufnahmeraum 10 aufgenommene und durch den Verformungsbereich 20 lagestabil festgelegte Produkt 4 zu allen Seiten sicher gegen Beaufschlagungen von außen, wie insbesondere gegen mechanische Beaufschlagungen, schützen zu können. Gleichzeitig wird durch die Anbringung des Schließelementes 22 auch der in der Vertiefung 26 aufgenommene Kragen 28 des Steckbereiches 18 dauerhaft gegenüber dem ersten Verpackungselement 6 gesichert.

Das Schließelement 22 und der Bundbereich 24 sind hierzu durch gegenseitig siegelfähige Kunststoffmaterialien gebildet. Beispielsweise kann hierbei der Bundbereich 24, beziehungsweise das zweite Verpackungselement 16 insgesamt, durch PET, PE oder PP und das Schließelement 22 durch eine Verbundfolie mit einer hierzu jeweils siegelfähigen, außen liegenden Schicht, oder vollständig aus einer PE-Folie oder aus medizinischem Papier gebildet sein, so dass die Verbindung allein durch Druck- und Temperaturbeaufschlagung hergestellt werden kann.

Darüber hinaus kann die Verbindung des Schließelementes 22 mit dem Bundbereich 24 peelfähig ausgebildet werden, um ein einfaches und komfortables Öffnen der fertig gestellten Verpackung zu ermöglichen. Hierzu kann zwischen dem Schließelement 22 und dem Bundbereich 24 eine zusätzliche Peelschicht 38 vorgesehen werden. Ferner kann beispielsweise am Schließelement 22 eine Peellasche 40 vorgesehen werden, um dieses leichter ergreifen und vom Bundbereich 24 trennen zu können.

Wie aus Fig. 4 zu entnehmen ist, kann am Aufnahmeboden 30 ferner eine Produktauflage 42 vorgesehen sein, die gegenüber dem übrigen Aufnahmeboden 30 erhöht ausgebildet ist und dadurch beim Einsetzen des zweiten Verpackungselementes 16 in den Aufnahmeraum 10 ein besseres Umhüllen des Produktes 4 mit dem jeweiligen erwärmten Verformungsbereich 20 gewährleistet. Auf diese Weise können auch relativ flache Produkte 4 sicher gegenüber dem ersten Verpackungselement 6 festgelegt werden.

Ferner ist es alternativ zu den in den Fig. 1 bis 4 dargestellten Ausführungsbeispielen der Erfindung möglich, in einer erfindungsgemäßen Verpackungsanordnung 2 nicht nur ein, sondern auch zwei oder mehrere Produkte 4 gleichzeitig aufzunehmen (nicht dargestellt).

In jedem Fall kann die aus der Verpackungsanordnung 2 hergestellte Verpackung mit dem darin sicher aufgenommenen und positionsstabil festgelegten wenigstens einen Produkt 4 anschließend einer Sterilisation unterzogen werden. Auf diese Weise steht das wenigstens eine Produkt 4 während und nach der Sterilisation in einem verpackten Zustand zur Verfügung, der bis zu dessen Verwendung eine geschützte und dichte Lagerung, Transportierbarkeit und Handhabung ermöglicht.

## Patentansprüche

1. Verpackung mit einer Verpackungsanordnung (2) und einem darin aufgenommenen sterilisierbaren Produkt (4),
wobei die Verpackungsanordnung (2) ein erstes Verpackungselement (6) mit einem Aufnahmeraum (10),
ein zweites Verpackungselement (16), das in den Aufnahmeraum (10) eingesetzt ist, und
ein Schließelement (22) aufweist, das unter Zwischenlage des zweiten Verpackungselementes (16) mit dem ersten Verpackungselement (6) verbunden ist,
wobei das zweite Verpackungselement (16) einen Verformungsbereich (20) aufweist, zwischen dem und einem Aufnahmeboden (30) des Aufnahmeraumes (10) ein Klemmbereich (34) für das Produkt (4) vorgesehen ist und der bei einer gegenüber einer üblichen Raumtemperatur erhöhten Verformungstemperatur zwischen 90 und 140° plastisch verformbar ist und das zweite Verpackungselement (16) einen sich um den Verformungsbereich (20) umlaufend erstreckenden und diesen aufspannenden Steckbereich (18) aufweist, der an einem Rand (12) des ersten Verpackungselementes (6) formschlüssig festgelegt ist,
wobei der Aufnahmeraum (10) durch wenigstens einen starren Tiefziehbereich (8) des ersten Verpackungselementes (6) gebildet ist und der Verformungsbereich (20) des zweiten Verpackungselementes (16) in einem Kontaktbereich (36) an dem Produkt (4) anliegt und komplementär zu diesem plastisch verformt ist.

2. Verpackung nach Anspruch 1, **dadurch gekennzeichnet, dass** das zweite Verpackungselement (16) blisterförmig ausgebildet ist.

3. Verpackung nach einem der Ansprüche 1 oder 2, **dadurch gekennzeichnet, dass** das Schließelement (22) und das erste Verpackungselement (6) wenigstens in dem miteinander verbindbaren Bereich durch gegenseitig siegelfähige Materialien gebildet sind.

4. Verpackung nach einem der Ansprüche 1 bis 3, **dadurch gekennzeichnet, dass** das erste Verpackungselement (6) und das Schließelement (22) eine peelbare Verbindung aufweisen.

5. Verpackung nach einem der Ansprüche 1 bis 4, **dadurch gekennzeichnet, dass** der Aufnahmeboden (30) eine Produktauflage (42) aufweist, die gegenüber dem übrigen Aufnahmeboden (30) erhöht ausgebildet ist.

6. Verfahren zur Herstellung einer Verpackung nach einem der Ansprüche 1 bis 5, **dadurch gekennzeichnet, dass**
in einem ersten Schritt das Produkt (4) im Aufnahmeraum (10) des ersten Verpackungselementes (6) angeordnet und das zweite Verpackungselement (16) auf die Verformungstemperatur erwärmt wird, bis der Verformungsbereich (20) einen plastisch verformbaren Zustand erreicht,
in einem zweiten Schritt das erwärmte zweite Verpackungselement (16) in den Aufnahmeraum (10) eingesetzt wird, wobei der Verformungsbereich (20) durch Anlage im Kontaktbereich (36) komplementär zum Produkt (4) ausgeformt wird und
in einem dritten Schritt eine Aufnahmeöffnung (14) des Aufnahmeraumes (10) mittels des Schließelementes (22) verschlossen wird.

7. Verfahren nach Anspruch 6, **dadurch gekennzeichnet, dass** im ersten Schritt lediglich der Verformungsbereich (20) auf die Verformungstemperatur erwärmt wird.

8. Verfahren nach Anspruch 6 oder 7, **dadurch gekennzeichnet, dass** die Verpackungsanordnung (2) mit dem darin festgelegten Produkt (4) in einem weiteren Schritt einer Sterilisation unterzogen wird.

## Claims

1. Packaging with a packaging arrangement (2) and a sterilisable product (4) accommodated therein,
wherein the packaging arrangement (2) comprises a first packaging element (6) with an accommodation space (10),
a second packaging element (16), which is located into the accommodation space (10), and a closure element (22), which is connected to the first packaging element (6), with the second packaging element (16) being located in between,
wherein the second packaging element (16) exhibits a deformation region (20), between which and an accommodation base (30) of the accommodation space (10) a clamping region (34) for the product (4) is provided, and which is plastically deformable at a deformation temperature increased above usual ambient temperature, of between 90 and 140°,
and the second packaging element (16) comprises an insertion region (18), extending circumferentially around the deformation region (20) and putting it under tension, which is secured in positive fit to an edge (12) of the first packaging element (6),
wherein the accommodation space (10) is formed by at least one rigid deep-drawn region (8) of the first packaging element (6), and the deformation region (20) of the second packaging element (16) is in contact with the product (4) in a contact region (36) and is plastically deformed in a way complementary to this.

2. Packaging according to claim 1, **characterised in that** the second packaging element (16) is configured in blister form.

3. Packaging according to any one of claims 1 or 2, **characterised in that** the closure element (22) and the first packaging element (6) are formed, at least in the region in which they can be connected to one another, by heat sealable materials.

4. Packaging according to any one of claims 1 to 3, **characterised in that** the first packaging element (6) and the closure element (22) comprise a peelable connection.

5. Packaging according to any one of claims 1 to 4, **characterised in that** the accommodation base (30) comprises a product support surface (42), which is configured as elevated in relation to the remainder of the accommodation base (30).

6. Method for producing a packaging according to any one of claims 1 to 5, **characterised in that**
in a first step, the product (4) is arranged in the accommodation space (10) of the first packaging element (6) and the second packaging element (16) is heated to the deformation temperature until the deformation region (20) attains a plastically deformable state,
in a second step, the heated second packaging element (16) is located in the accommodation space (10), wherein the deformation region (20) is shaped by contact in the contact region (36) in a manner complementary to the product (4), and
in a third step, an accommodation opening (14) of the accommodation space (10) is closed by means of the closure element (22).

7. Method according to claim 6, **characterised in that** in the first step only the deformation region (20) is heated to the deformation temperature.

8. Method according to claim 6 or 7, **characterised in that** the packaging arrangement (2), with the product (4) secured in it, is subjected to a sterilisation in a further step.

## Revendications

1. Emballage comprenant un ensemble d'emballage (2) et un produit (4) pouvant être stérilisé logé dans ce dernier,
dans lequel l'ensemble d'emballage (2) comprend un premier élément d'emballage (6) présentant un logement de réception (10),
un deuxième élément d'emballage (16) inséré dans le logement de réception (10), et
un élément de fermeture (22) relié avec le premier élément de d'emballage (6) par l'intermédiaire d'une couche intermédiaire du deuxième élément d'emballage (16),
dans lequel le deuxième élément d'emballage (16) comprend une zone de déformation (20), entre laquelle et un fond de réception (30) du logement de réception (10) il est prévu une zone de blocage (34) pour le produit (4) et la zone de déformation peut être déformée plastiquement par une température de déformation entre 90 et 140°, augmentée par rapport à une température ordinaire de logement,
et le deuxième élément d'emballage (16) comprend une zone de raccordement (18) s'étendant circonférentiellement autour de la zone de déformation (20) et montée à cette dernière, et fixée par complémentarité de forme sur une bordure (12) du premier élément d'emballage (6),
dans lequel le logement de réception (10) est réalisé par au moins une zone thermoformée (8) rigide du premier élément d'emballage (6) et la zone de déformation (20) du deuxième élément d'emballage (16) est posée dans une zone de contact (36) sur le produit (4) et est déformée plastiquement par complémentarité de forme à ce dernier.

2. Emballage selon la revendication 1, **caractérisé en ce que** le deuxième élément d'emballage (16) est réalisé en habillage transparent.

3. Emballage selon la revendication 1 ou 2, **caractérisé en ce que** l'élément de fermeture (22) et le premier élément d'emballage (6) sont réalisés au moins dans la zone de raccordement réciproque par des matériaux scellables réciproquement.

4. Emballage selon l'une des revendications 1 à 3, **caractérisé en ce que** le premier élément d'emballage (6) et l'élément de fermeture (22) comprennent un raccordement pelable.

5. Emballage selon l'une des revendications 1 à 4, **caractérisé en ce que** le fond de réception (30) comprend un support de produit (42) qui est réalisé de manière surélevée par rapport au reste du fond de réception (30).

6. Procédé de fabrication d'un emballage selon l'une des revendications 1 à 5, **caractérisé en ce que**
dans une première étape le produit (4) est disposé dans le logement de réception (10) du premier élément d'emballage (6) et le deuxième élément d'emballage (16) est échauffé à la température de déformation jusqu'à ce que la zone de déformation (20) atteigne un état pouvant être déformé plastiquement,
dans une deuxième étape le deuxième élément d'emballage (16) échauffé est inséré dans le logement de réception (10), la zone de déformation (20) étant formée au produit (4) par complémentarité de forme par application dans la zone de contact (36), et
dans une troisième étape une ouverture de réception (14) du logement de réception (10) est fermée au moyen de l'élément de fermeture (22).

7. Procédé selon la revendication 6, **caractérisé en ce que,** dans la première étape, seule la zone de déformation (20) est échauffée à la température de déformation.

8. Procédé selon la revendication 6 ou 7, **caractérisé en ce que** l'ensemble d'emballage (2), comprenant le produit (4) fixé dans ce dernier, est soumis à une stérilisation dans une étape supplémentaire.
